# EUROPEAN PATENT APPLICATION

(11) **EP 1 998 167 A2**
(43) Date of publication of application: **03.12.2008**
(21) Application number: 08151362.4
(22) Date of filing: 13.02.2008
(51) Int. Cl.: G01N 21/64

(54) **Fluorescence detecting module for microreaction and fluorescence detecting system having the same**

(30) Priority: 01.06.2007 KR 20070054023
(71) Applicant: Samsung Electronics Co., Ltd., Suwon-si, Gyeonggi-Do (KR)
(72) Inventor: Kim, Su-hyeon c/o Samsung Advanced Institute of Technology, Gyeonggi-do (KR); KIM, Jin-tae c/o Samsung Advanced Institute of Technology, Gyeonggi-do (KR)
(74) Representative: Grünecker, Kinkeldey, Stockmair & Schwanhäusser Anwaltssozietät

(57) **Abstract**

Provided are a fluorescence detecting module for detecting fluorescence in a microchamber and a fluorescence detecting system having the same. The fluorescence detecting module includes a light source irradiating excitation light, a collimating lens condensing excitation light irradiated from the light source, a dichroic mirror selectively transmitting or reflecting the light according to its wavelength, an objective lens condensing excitation light selected by the dichroic mirror to be irradiated on the sample in a microchamber and condensing fluorescence generated in the microchamber, a focusing lens focusing fluorescence selected by the dichroic mirror, and a fluorescence detecting element detecting fluorescence focused by the focusing lens. The fluorescence detecting system for a microfluid chip in which a plurality of microchambers are arranged includes a frame, at least one fluorescence detecting module, a holder supporting the at least one fluorescence detecting module, a driver installed in the frame and allowing the holder to make a reciprocating motion along a direction in which the plurality of microchambers are arranged, and a guide installed in the frame, supporting the holder to be moved and guiding the movement.

## Description

### BACKGROUND OF THE INVENTION

### 1. Field of the Invention

The present invention relates to microfluidics, and more particularly, to a fluorescence detecting module for detecting fluorescence in a microchamber in which a microreaction occurs, and a fluorescence detecting system having the same.

### 2. Description of the Related Art

Microfludics are techniques in which a microchamber is formed on a chip using micromachining technology such as photolithography, hot-embossing or molding and the reaction of microfluid occurs in the microchamber. Microfluidics have advantages that the amount of a consumed reagent can be reduced and an analysis time can be reduced. The microchamber is a space in which a microfluid to be analyzed is kept. A microchannel is connected to the microchamber and the microchamber has a width that is larger or equal to the width of a microchannel having several tens to several hundreds of micrometers. A microreaction in the microchamber usually accompanies a biochemical reaction such as polymerase chain reaction (PCR), enzyme reaction or immunoassay etc. In order to analyze the microreaction, fluorescence generated in the microchamber is detected.

In particular, in the case where different temperatures are required in denaturation, annealing, and extension like in PCR, a temperature cycle is repeatedly applied so that a reaction can be performed. At this time, due to a small reaction volume and a wide area, heat is rapidly transmitted to the microchamber so that a time required for the temperature cycle can be reduced.

There are several methods for detecting a PCR in real time. However, a fluorescence detecting method is used in most commercialized apparatuses. A variety of fluorescence detecting methods have been developed. Such methods include a method of using a fluorescence dye such as SYBR Green I for generating fluorescence by combining with a double strand DNA generated by PCR and a TaqMan(R) method in which a DNA sequence that can be combined between two primers used in PCR is used as a probe, fluorophore and quencher are combined at both ends of the probe, then if the probe is cut using exonuclease activity of Taq polymerase used in DNA synthesis, the fluorophore and the quencher are separated from each other and fluorescence occurs.

Currently, a biochemical reaction such as PCR is usually performed in a tube and various apparatuses for detecting fluorescence in the tube has been commercialized.

U.S. Patent No. 5,928,907 of Applied Biosystems which has been granted discloses an apparatus for detecting fluorescence in a tube using optical fiber. The apparatus is advantageous in that a plurality of tubes can be detected by one detector. However, in order to condense excitation light for exciting fluorescence in the optical fiber, a well-collimated light source like a laser must be used. In addition, a precise optical apparatus is needed and thus the apparatus can be applied only to equipment having high throughput.

An apparatus disclosed in U.S. Patent No. 6,369,893 of Cepheid which has been granted is constituted by comprising an excitation block and a detection block. Fluorescence is excited using the excitation block using an LED and a fluorescence signal is detected using the detection block positioned at 90 degrees and thus, the apparatus is advantageous to modulation. However, in order to perform excitation and detection at 90 degrees, a tube is formed to have a diamond shape and excitation and detection is performed two thin walls. Thus, since a sufficient space between the walls is needed, a sample volume of 25µℓ or more is needed.

U.S. Patent No. 7,081,226 of Idaho Technology which has been granted discloses a method of using a capillary tube as a PCR reaction container. In the apparatus, an LED light source is collimated and is irradiated into the capillary tube through a lens, fluorescence generated in the tube is condensed on the same lens and is selectively reflected at 90 degrees using a dichroic mirror and the reflected fluorescence is detected. The apparatus is appropriate for a reaction container having a small diameter like the capillary tube but is not appropriate for a reaction container having a smaller thickness and larger area like a microfluid chip.

In an apparatus disclosed in U.S. Patent No. 7,148,043 of MJ Research which has been granted, a conventional well-structured thermal cycle is used, an LED light source is irradiated on a well and fluorescence is condensed and detected. The apparatus can detect a reaction solution having a volume of several tens of µℓ like a 96 or 384 well plate. However, in order to detect a reaction which occurs in a microchamber having a volume of less than several µℓ and a small depth of less than 500 µm, the size of a light source irradiated into the microchamber must be small and a focus distance of an optical system must be precisely maintained. Thus, the apparatus is not appropriate for detecting a reaction in the microchamber.

A conventional PCR reaction device is a large table top-shaped device and in general, a plastic well or tube is used as a reaction container and a very large thermal mass is used as a heating means like an aluminum block. Thus, the conventional PCR reaction device is inefficient as a heating and cooling speed is slow and power consumption is high.

Thus, a technology of using a microfluid chip in which a microchamber of which volume is minimized on a substrate formed of silicon or a silicon-based material having thermal conductivity as a reaction container is formed has been developed. In order to improve throughput, a plurality of microchambers are formed in the microfluid chip. Therefore, as a distance between microchambers is narrower than a distance between wells in a conventional well plate, many microreactions can be accepted per unit area. Thus, the technology is advantageous.

However, a fluorescence detector for detecting a microreaction that occurs in microchambers having a narrow distance generally uses a laser light source. In general, the laser light source having a wavelength used in fluorescence detection has a large size and a method of using optical fiber is used as a method for connecting a light source to a driving optical system. In this case, precise optical components are needed for coupling of a light source and an optical fiber and costs are increased.

### SUMMARY OF THE INVENTION

The present invention provides a fluorescence detecting module having an optical system that is appropriate for detecting of fluorescence in a plurality of microchambers of a microfluid chip and a fluorescence detecting system having the same.

According to an embodiment of the present invention, there is provided a fluorescence detecting module comprising: a light source irradiating excitation light; a collimating lens condensing excitation light irradiated from the light source; a dichroic mirror selectively transmitting or reflecting the light according to its wavelength; an objective lens condensing excitation light selected by the dichroic mirror to be irradiated on the sample in a microchamber and condensing fluorescence generated in the microchamber; a focusing lens focusing fluorescence selected by the dichroic mirror; and a fluorescence detecting element detecting fluorescence focused by the focusing lens.

The light source may be a light emitting diode (LED) having a surface emission shaped LED chip, and an emission surface of the LED chip may be projected onto a sample in the microchamber as an optical spot having a predetermined area. The ratio of the area of the optical spot to the area of the emission surface of the LED chip may be 1 or less than 1. The optical spot may be positioned in the microchamber. The optical spot may be positioned at the middle of the depth of the microchamber. The emission surface of the LED chip may have a shape that is long in the lengthwise direction of the microchamber. The LED may be an LED having no lens.

The collimating lens may condense excitation light into substantially parallel light.

The dichroic mirror may be disposed to be inclined at approximately 45 degrees with respect to an optical axis of excitation light irradiated from the light source and selectively transmitting, or reflecting at right angles, excitation light and fluorescence according to their wavelengths.

The dichroic mirror may reflect short-wavelength components of excitation light at right angles to be directed toward the objective lens and transmit long-wavelength components of the fluorescence to be directed toward the focusing lens.

The dichroic mirror may transmit short-wavelength components of excitation light to be directed toward the objective lens and reflects long-wavelength components of the fluorescence at right angles to be directed toward the focusing lens.

The fluorescence detecting element may be a photo diode having an active region or an Avalanche photo diode having an amplification capability.

The fluorescence detecting module may further comprise: a first filter disposed between the collimating lens and the dichroic mirror and selecting a wavelength of excitation light; and a second filter disposed between the dichroic mirror and the focusing lens and selecting a wavelength of fluorescence. The first filter may be disposed at right angles with respect to an optical axis of excitation light irradiated from the light source, and the second filter may be disposed at right angles with respect to an optical axis of fluorescence that is directed towards the fluorescence detecting element. The first filter may be a short-wavelength transmission filter transmitting short-wavelength components of excitation light, and the second filter may be a long-wavelength transmission filter transmitting long-wavelength components of fluorescence. The first filter and the second filter may be dichroic filters.

The fluorescence detecting module may further comprise a base in which a first optical path, a second optical path, and a third optical path connected to one another are formed, and excitation light irradiated from the light source may be projected onto a sample in the microchamber through the first optical path and the second optical path, and fluorescence generated in the microchamber may reach the fluorescence detecting element through the second optical path and the third optical path.

The light source may be installed at an end of the first optical path, the objective lens may be installed at an end of the second optical path, the fluorescence detecting element may be installed at an end of the third optical path, the collimating lens is installed within the first optical path, and the focusing lens may be installed within the third optical path, and the dichroic mirror may be inserted and installed in a position in which the first optical path, the second optical path, and the third optical path meet one another to be inclined at approximately 45 degrees with respect to the optical axis of excitation light irradiated from the light source.

The second optical path and the third optical path may be parallel to each other in a vertical direction and the first optical path may be formed in a horizontal direction and meet the second optical path and the third optical path at right angles. The dichroic mirror may reflect short-wavelength components of excitation light that has passed through the first optical path at right angles to be directed toward the objective lens through the second optical path, and the dichroic mirror may transmit long-wavelength components of fluorescence that is generated in the microchamber and has passed through the second optical path to be directed toward the focusing lens through the third optical path.

The first optical path and the second optical path may be parallel to each other in a vertical direction and the third optical path is formed in a horizontal direction and meet the first optical path and the second optical path at right angles. The dichroic mirror may transmit short-wavelength components of excitation light that has passed through the first optical path to be directed toward the objective lens through the second optical path, and the dichroic mirror may reflect long-wavelength components of fluorescence that are generated in the microchamber and that have passed through the second optical path at right angles to be directed toward the focusing lens through the third optical path.

A first filter that selects a wavelength of excitation light between the collimating lens and the dichroic mirror may be further installed in the first optical path, and a second filter that selects a wavelength of fluorescence between the focusing lens and the dichroic mirror may be further installed in the third optical path.

According to another embodiment of the present invention, there is provided a fluorescence detecting system for a microfluid chip in which a plurality of microchambers are arranged, the system comprising: a frame; at least one fluorescence detecting module detecting fluorescence in the microchamber; a holder supporting the at least one fluorescence detecting module; a driver installed in the frame and allowing the holder to make a reciprocating motion along a direction in which the plurality of microchambers are arranged; and a guide installed in the frame, supporting the holder to be moved and guiding the movement.

A plurality of fluorescence detecting modules arranged in the same direction as the arrangement direction of the plurality of microchambers may be installed in the holder. The plurality of fluorescence detecting modules may detect at least two kinds of fluorescence having different wavelengths. Each of the plurality of fluorescence detecting modules may irradiate excitation light having different wavelengths and detect fluorescence having different wavelengths.

The driver may comprise a lead screw combined with the holder and a driving motor rotating the lead screw.

The guide may be long in the movement direction of the holder and support upper and lower portions of the holder.

### BRIEF DESCRIPTION OF THE DRAWINGS

The above and other features and advantages of the present invention will become more apparent by describing in detail exemplary embodiments thereof with reference to the attached drawings in which:
FIG. 1A is a perspective view of a traditional microfluid chip used in a fluorescence detecting system according to an embodiment of the present invention;
FIG. 1B is a cross-sectional view of a microfluid chip of FIG. 1A taken along line A-A';
FIG. 2 schematically illustrates a structure of a fluorescence detecting module according to an embodiment of the present invention;
FIG. 3 schematically illustrates a structure of a fluorescence detecting module according to another embodiment of the present invention;
FIG. 4 is a plan view showing an optical spot of excitation light irradiated on a sample in a microchamber using the fluorescence detecting module of FIG. 2 or 3;
FIG. 5 shows a transmission spectrum according to an incidence angle of light that is incident on a dichroic mirror;
FIG. 6 is a perspective view of a specific structure of the fluorescence detecting module of FIG. 2;
FIG. 7 is a perspective view of a specific structure of the fluorescence detecting module of FIG. 3;
FIG. 8 is a perspective view of a fluorescence detecting system according to an embodiment of the present invention;
FIG. 9 shows wavelength spectrums of LEDs installed in six fluorescence detecting modules mounted in the fluorescence detecting system of FIG. 8 for experiments;
FIGS. 10A and 10B show excitation spectrums and fluorescence spectrums of fluorescence dyes injected into microchambers of a microfluid cip for experiments; and
FIG. 11 shows fluorescence spectrums detected by the fluorescence detecting module according to the present invention as a result of experiments.

### DETAILED DESCRIPTION OF THE INVENTION

The present invention will now be described more fully with reference to the accompanying drawings, in which exemplary embodiments of the invention are shown. In the following drawings, like reference numerals denote like elements.

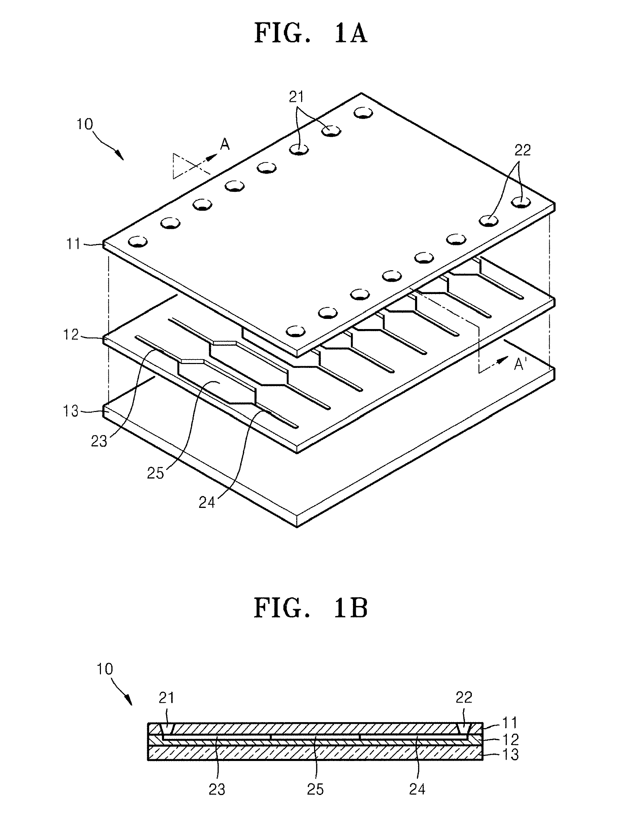

FIG. 1A is a perspective view of a traditional microfluid chip used in a fluorescence detecting system according to an embodiment of the present invention, and FIG. 1B is a cross-sectional view of the microfluid chip of FIG. 1A taken along line A-A'.

Referring to FIGS. 1A and 1B, a microfluid chip 10 comprises an upper substrate 11 in which a sample inlet 21 and a sample outlet 22 are formed, a lower substrate 12 in which a microchamber 25 and a microchannel 24 in which a microreaction occurs are formed, and a heater 13 which adjusts a reaction temperature in the microchamber 25.

The lower substrate 12 may be formed of silicon, metal or plastics having a high thermal conductivity efficiency so as to facilitate heat transfer from the heater 13, and the upper substrate 11 may be formed of a transparent material such as glass or transparent plastics so as to facilitate fluorescence detection. The upper substrate 11 and the lower substrate 12 may be bonded to each other using anodic bonding, thermal bonding or bonding using an adhesive. The sample inlet 21, the sample outlet 22, and the microchamber 25 and the microchannels 23 and 24 may be formed using a method such as photolithography, hot-embossing, blasting or plastic molding.

The microfluid chip 10 may have a plurality of microchambers 25 and a plurality of microchannels 23 and 24 so as to detect various reactions with respect to a variety of types of samples or one type of sample. As described above, in the microfluid chip 10 having the plurality of microchambers 25 and the plurality of microchannels 23 and 24, the plurality of microchambers 25 may be arranged one-dimensionally, that is, in along one direction. This is because, unlike a conventional well plate, for example, unlike a microtiter plate, when the microchambers 25 are arranged two-dimensionally, the microchannels 23 and 24 must pass between the microchambers 24 and thus, a structure of a microfluid chip becomes complicated.

As described above, when the microchambers 25 are arranged one-dimensionally, the width of the microchambers 25 may be larger than that of the microchannels 23 and 24 so that an area detected with respect to an incoming sample can be maximized. In order to arrange a plurality of microchambers 25 having as large as number in a predetermined area, it is preferable that a distance between microchambers 25 is gradually reduced. As such, the width of the microchambers 25 may be less than 1.5mm and a distance between centers of the microchambers 25 may be less than approximately 2mm.

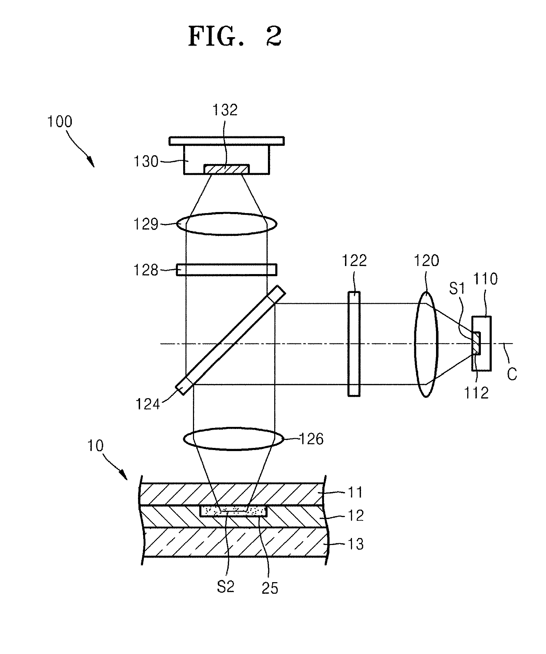

FIG. 2 schematically illustrates a structure of a fluorescence detecting module according to an embodiment of the present invention.

Referring to FIG. 2, a fluorescence detecting module 100 according to an embodiment of the present invention comprises a light source 110, a collimating lens 120, a dichroic mirror 124, an objective lens 126, a focusing lens 129, and a fluorescence detecting element 130.

Specifically, the light source 110 irradiates excitation light used to excite fluorescence. The collimating lens 120 is disposed in front of the light source 110, for example, a light emitting diode (LED) 110 and condenses excitation light that is irradiated at a predetermined angle from the light source 110 into substantially parallel light. The dichroic mirror 124 is disposed to be inclined at 45 degrees with respect to an optical axis C of excitation light. The dichroic mirror 124 transmits long-wavelength components of excitation light that are similar to a fluorescence wavelength and reflects short-wavelength components of excitation light that pass through the collimating lens 120 at right angles. Excitation light reflected by the dichroic mirror 124 is condensed by the objective lens 126 and is irradiated on a sample in the microchamber 25 of the microfluid chip 10.

Fluorescence that is generated in the microchamber 25 by irradiating excitation light is condensed by the objective lens 126 to approximately parallel light. Fluorescence that is condensed by the objective lens 126 transmits the dichroic mirror 124 which transmits long-wavelength components as described above. Fluorescence that has transmitted the dichroic mirror 124 is focused by the focusing lens 129 and is irradiated on the fluorescence detecting element 130, for example, a photo diode 130 having an active region 132. The photo diode 130 generates an electrical signal corresponding to received fluorescence from the received fluorescence. An Avalanche photo diode having an amplification capability may also be used as the fluorescence detecting element 130.

The fluorescence detecting module 100 according to the above-described embodiment of the present invention may further comprise a first filter 122 and a second filter 128. The first filter 122 is disposed between the collimating lens 120 and the dichroic mirror 124, and a short-wavelength transmission filter which reflects long-wavelength components of excitation light that are similar to fluorescence wavelength and transmits short-wavelength components of excitation light, is used as the first filter 122. The second filter 128 is disposed between the dichroic mirror 124 and the focusing lens 129. A long-wavelength transmission filter which transmits long-wavelength fluorescence and reflects short-wavelength excitation light which may act as a background signal is used as the second filter 128. Long-wavelength components that are similar to fluorescence light irradiated on the sample in the microchamber 25 may be minimized by the first filter 122, and short-wavelength excitation light which is included in fluorescence irradiated on the photo diode 130 and may act as a background signal may be minimized by the second filter 128.

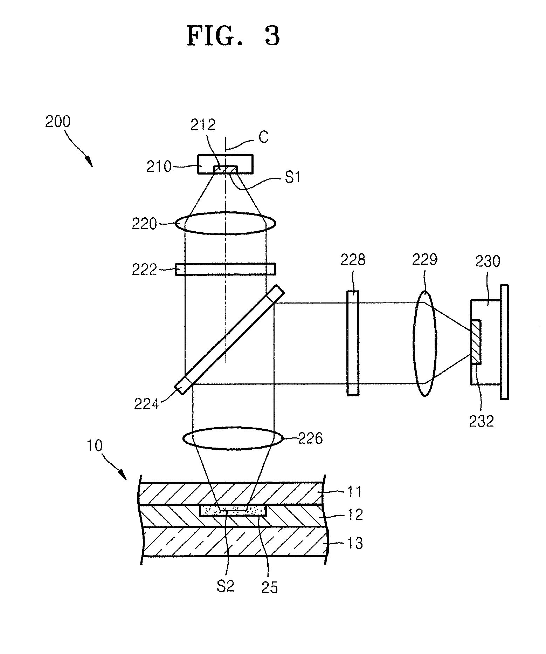

FIG. 3 schematically illustrates a structure of a fluorescence detecting module according to another embodiment of the present invention. Referring to FIG. 3, a fluorescence detecting module 200 according to another embodiment of the present invention comprises a light source 210, a collimating lens 220, a dichroic mirror 224, an objective lens 226, a focusing lens 229, and a fluorescence detecting element 230.

Specifically, the light source 210 irradiates excitation light used to excite fluorescence. The collimating lens 220 is disposed in front of the light source 210 and condenses excitation light that is irradiated at a predetermined angle from the light source 210 into substantially parallel light. The dichroic mirror 224 is disposed to be inclined at 45 degrees with respect to an optical axis C of excitation light. The dichroic mirror 224 reflects long-wavelength components of excitation light that are similar to a fluorescence wavelength and transmits short-wavelength components of excitation light that passes through the collimating lens 220. Excitation light that has transmitted the dichroic mirror 224 is condensed by the objective lens 226 and is irradiated on a sample in the microchamber 25 of the microfluid chip 10.

Fluorescence that is generated in the microchamber 25 by irradiation of excitation light is condensed by the objective lens 226 into approximately parallel light. Fluorescence that is condensed by the objective lens 226 is reflected by the dichroic mirror 224 which reflects long-wavelength components as described above at right angles. Fluorescence that is reflected by the dichroic mirror 224 is focused by the focusing lens 229 and is irradiated on the fluorescence detecting element 230, for example, a photo diode 230 having an active region 232. The photo diode 230 generates an electrical signal corresponding to received fluorescence from the received fluorescence.

The fluorescence detecting module 200 according to the above-described embodiment of the present invention may further comprise a first filter 222 and a second filter 228. The first filter 222 is disposed between the collimating lens 220 and the dichroic mirror 224, and a short-wavelength transmission filter which reflects long-wavelength components of excitation light that are similar to fluorescence wavelength and transmits short-wavelength components of excitation light, is used as the first filter 222. The second filter 228 is disposed between the dichroic mirror 224 and the focusing lens 229. A long-wavelength transmission filter which transmits long-wavelength fluorescence and reflects short-wavelength excitation light which may act as a background signal is used as the second filter 228. Long-wavelength components that are similar to fluorescence light irradiated on the sample in the microchamber 25 may be minimized by the first filter 222, and short-wavelength excitation light which is included in fluorescence irradiated on the photo diode 230 and may act as a background signal may be minimized by the second filter 228.

In the fluorescence detecting modules 100 and 200 shown in FIGS. 2 and 3, LEDs each having surface emission shaped LED chips 112 and 212 may be used as the light sources 110 and 210. Thus, an emission surface S1 of the LED chips 112 and 212 of the LEDs 110 and 210 is projected onto the sample in the microchamber 25 as an optical spot S2 having a predetermined area. In this case, even if a distance between the microchambers 25 is narrow, so as not to affect the adjacent microchambers 25, the area of the optical spot S2 of excitation light irradiated on the sample in the microchamber 25 may be equal to or smaller than the area of the emission surface S1 of the LED chips 112 and 212. In other words, the ratio of the area of the optical spot S2 of excitation light to the area of the emission surface S1 of the LED chips 112 and 212 may be 1 or less than 1. The optical spot S2 of excitation light may be positioned inside the microchamber 25 and more preferably, may be positioned approximately at the middle of the depth of the microchamber 25. The area and position of the optical spot S2 of excitation light may be controlled by the collimating lenses 120 and 220 and the objective lenses 126 and 226.

The width of the LED chips 112 and 212 is generally more than 0.2 mm. Thus, even when the ratio is 1, the width of the optical spot S2 is more than 0.2 mm. Thus, the distance between the microchambers 25 may be more than 0.2 mm. As described above, in order to arrange a large number of microchambers 25 in a predetermined area, the distance between the microchambers 25 may be less than 2 mm.

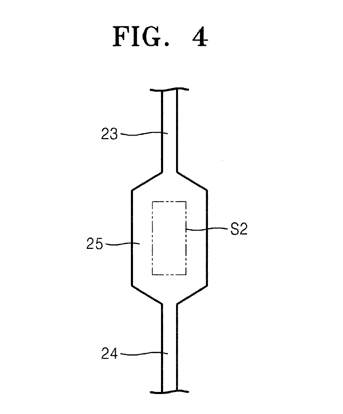

The quantity of excitation light irradiated from the LED chips 112 and 212 increases as the area of the emission surface S1 increases. As such, the area of the optical spot S2 of excitation light irradiated on the sample in the microchamber 25 also increases and fluorescence can be more efficiently generated. However, as described above, the width of the optical spot S2 must be limited so that excitation light irradiated on the sample in a microchamber 25 does not affect the other adjacent microchambers 25. In order to satisfy the limitation and increase the area of the optical spot S2, as illustrated in FIG. 4, an optical spot S2 that is long in the lengthwise direction of the microchamber 25 may be formed. Thus, the LED chips 112 and 212 may have an emission surface S1 that is long in the lengthwise direction of the microchamber 25.

A general LED is provided to have a shape in which an LED chip is molded in transparent plastic. A structure in which transparent plastic are made to have a shape that acts as a lens and the irradiation angle of light irradiated from the LED chip is reduced is usually used as an LED. However, in this case, due to an error in a manufacturing process, a difference in positions of the LED chips molded in plastics may occur. As such, irradiation patterns may be changed. Thus, the LEDs 110 and 210 each having no lens may be used in the present invention.

In the fluorescence detecting modules 100 and 200 shown in FIGS. 2 and 3, as described above, a short-wavelength transmission filter is used as the first filters 122 and 222 and a long-wavelength transmission filter is used as the second filters 128 and 228. Specifically, a dichroic filter may be used as the first filters 122 and 222 and the second filters 128 and 228. The dichroic filter is a very sophisticated color filter, has a structure in which coating layers having different refractive indices are sequentially formed on a glass substrate and has a characteristic that light having a particular wavelength is transmitted and light having a different wavelength is reflected. In embodiments of the present invention, a dichroic filter in which transmission and reflection wavelengths are decided with respect to light having an incidence angle of 0 degree has been used as the first filters 122 and 222 and the second filters 128 and 228. As described above, the dichroic filter may be manufactured of a long-wavelength transmission filter or a short-wavelength transmission filter. In addition, a combination of a short-wavelength transmission filter and a long-wavelength transmission filter may be used so as to transmit light having a particular wavelength.

As illustrated in FIGS. 2 and 3, the dichroic mirrors 124 and 224 have the same structure as the above-described dichroic filter. However, the dichoric mirrors 124 and 224 have a characteristic in which transmission and reflection wavelengths are decided with respect to light having an incidence angle of 45 degrees. In this way, the dichoric mirrors 124 and 224 are affected by an incidence angle of light.

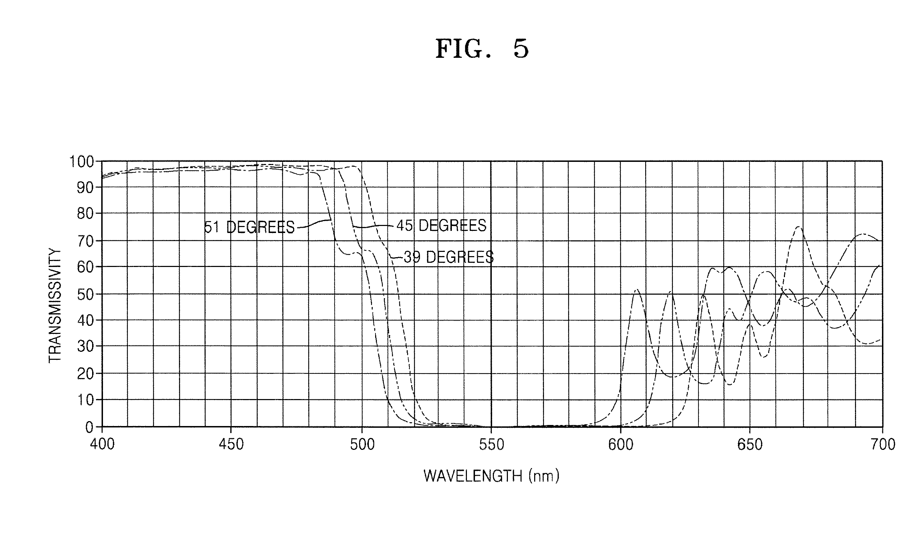

FIG. 5 shows a transmission spectrum according to an incidence angle of light that is incident on a dichroic mirror. Referring to FIG. 5, when the incidence angle of light is varied from 45 degrees by ±6 degrees, a transmission wavelength varies by approximately ± 5 nm. Thus, as light having various incidence angles passes through the dichroic mirror, a filtering effect is lowered.

Thus, in the present invention, light of which incidence angle is near 45 degrees needs to pass through the dichroic mirrors 124 and 224 as much quantity as possible. Thus, the distance between the LEDs 110 and 210 and the collimating lenses 120 and 220 may be designed so that excitation light irradiated from the LEDs 110 and 210 can be condensed by the collimating lenses 120 and 220 to be as near to parallel light as possible.

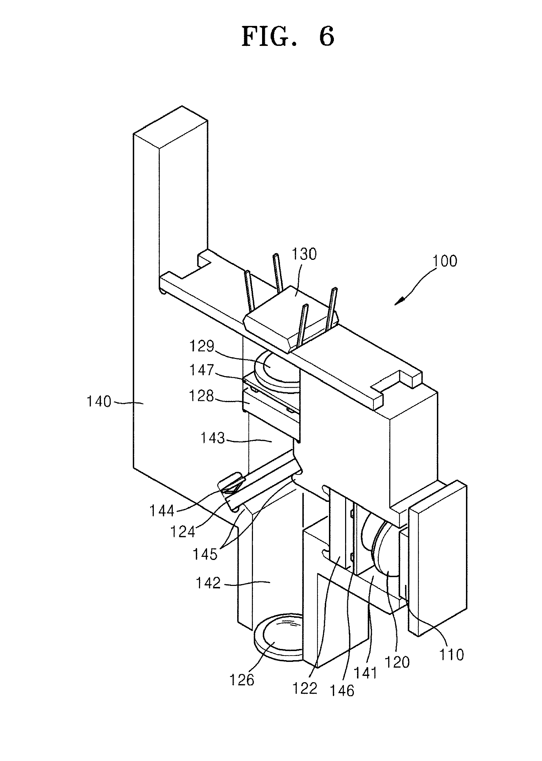

FIG. 6 is a perspective view of a specific structure of the fluorescence detecting module of FIG. 2.

Referring to FIG. 6, a fluorescence detecting module 100 according to an embodiment of the present invention may comprise a base 140. A first optical path 141, a second optical path 142, and a third optical path 143 which are connected to one another, are formed in the base 140. The LED 110 is installed at an end of the first optical path 141, the objective lens 126 is installed at an end of the second optical path 142, and the photo diode 130 is installed at an end of the third optical path 143. The second optical path 142 and the third optical path 143 are parallel to each other in a vertical direction and the first optical path 141 is formed in a horizontal direction and meets the second optical path 142 and the third optical path 143 at right angles. Excitation light that is irradiated from the LED 110 is irradiated on the sample in the microchamber 25 of the microfluid chip 10 through the first optical path 141 and the second optical path 142, and fluorescence generated in the microchamber 25 passes through the second optical path 142 and the third optical path 143 and reaches the photo diode 130.

The dichroic mirror 124 is inserted and installed at the position in which the first optical path 141, the second optical path 142, and the third optical path 143 meet one another, to be inclined at 45 degrees with respect to the optical axis of excitation light that is irradiated from the LED 110. The dichroic mirror 124 may be fixed by a mirror fixing spring 144 and a mirror support jaw 145 in a correct position at an accurate angle. An adhesive may be additionally used to more firmly fix the dichroic mirror 124. The collimating lens 120 is installed in the first optical path 141 at right angles with respect to the optical axis of excitation light, and the focusing lens 129 is installed in the third optical path 143 at right angles with respect to the optical axis of fluorescence that is directed toward the photo diode 130. The first filter 122 is inserted and installed in the first optical path 141 between the collimating lens 120 and the dichroic mirror 124 at right angles with respect to the optical axis of excitation light, and the second filter 128 is inserted and installed in the third optical path 143 between the focusing lens 129 and the dichroic mirror 124 at right angles with respect to the optical axis of fluorescence. The first filter 122 and the second filter 128 may be fixed by filter fixing springs 146 and 147.

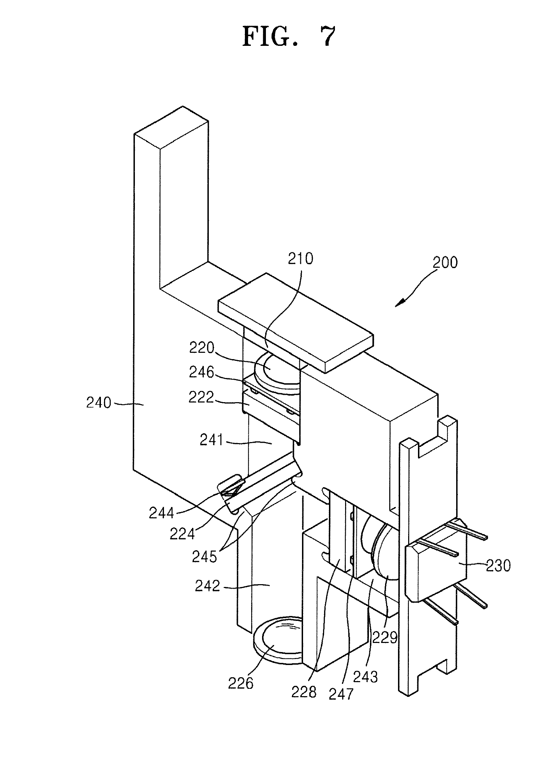

FIG. 7 is a perspective view of a specific structure of the fluorescence detecting module of FIG. 3.

Referring to FIG. 7, the fluorescence detecting module 200 according to another embodiment of the present invention may further comprise a base 240. A first optical path 241, a second optical path 242, and a third optical path 243 are formed in the base 240. The LED 210 is installed at the end of the first optical path 241, the objective lens 226 is installed at the end of the second optical path 242, and the photo diode 230 is installed at the end of the third optical path 243. The first optical path 241 and the second optical path 242 are formed to be parallel to each other in a vertical direction, and the third optical path 243 is formed in a horizontal direction and meets the first optical path 241 and the second optical path 242 at right angles. Excitation light that is irradiated from the LED 210 is irradiated on the sample in the microchamber 25 of the microfluid chip 10 through the first optical path 241 and the second optical path 242, and fluorescence that is generated in the microchamber 25 passes along the second optical path 242 and the third optical path 243 and reaches the photo diode 230.

The dichroic mirror 224 is inserted and installed at the position in which the first optical path 241, the second optical path 242, and the third optical path 243 meet one another, to be inclined at 45 degrees with respect to the optical axis of excitation light that is irradiated from the LED 210. The dichroic mirror 224 may be fixed by a mirror fixing spring 244 and a mirror support jaw 245 in a correct position at an accurate angle. An adhesive may be additionally used to more firmly fix the dichroic mirror 224. The collimating lens 220 is installed in the first optical path 241 at right angles with respect to the optical axis of excitation light, and the focusing lens 229 is installed on the third optical path 243 at right angles with respect to the optical axis of fluorescence that is directed toward the photo diode 230. The first filter 222 is inserted and installed on the first optical path 241 between the collimating lens 220 and the dichroic mirror 224 at right angles with respect to the optical axis of excitation light, and the second filter 228 is inserted and installed on the third optical path 243 between the focusing lens 229 and the dichroic mirror 224 at right angles with respect to the optical axis of fluorescence. The first filter 222 and the second filter 228 may be fixed by filter fixing springs 246 and 247.

In the fluorescence detecting modules 100 and 200 shown in FIGS. 6 and 7, the LEDs 110 and 210, the dichroic mirrors 124 and 224, the first filters 122 and 222, and the second filters 128 and 228 may be selected according to wavelengths to be detected. All of the collimating lenses 120 and 220, the objective lenses 126 and 226, and the focusing lenses 129 and 229 may be lenses having clear apertures of less than 4 mm.

Optical components of the fluorescence detecting modules 100 and 200 may be assembled on the same bases 140 and 240 regardless of wavelengths to be detected. Only, in order to improve a condensing efficiency according to wavelengths, a distance between the collimating lenses 120 and 220 and the LEDs 110 and 210 may be slightly modified within the range of approximately 0.1 mm. However, other optical components may be used without adjustment of installation positions even when wavelengths to be detected are changed.

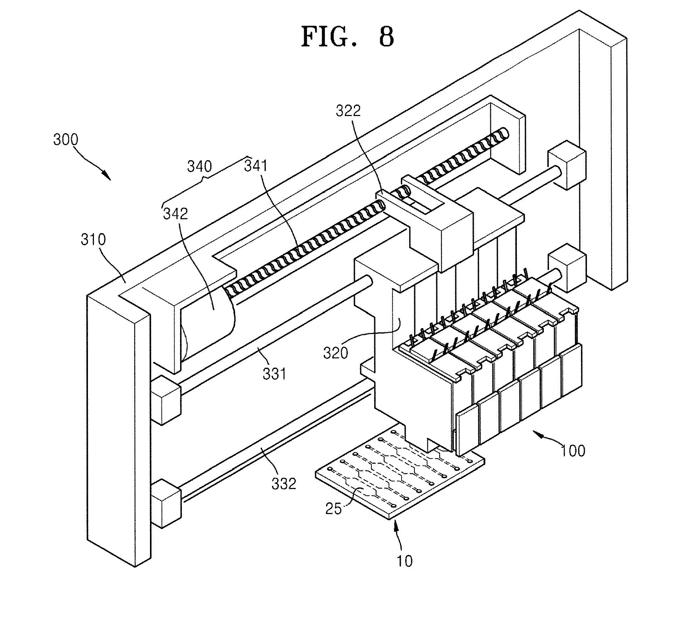

FIG. 8 is a perspective view of a fluorescence detecting system according to an embodiment of the present invention.

Referring to FIG. 8, a fluorescence detecting system 300 according to the present invention has a structure in which the fluorescence detecting system 300 makes a reciprocating motion in a direction in which the microchambers 25 of the microfluid chip 10 are arranged and detects fluorescence in the microchambers 25. To this end, the fluorescence detecting system 300 according to the present invention comprises a frame 310, a holder 320 which supports at least one fluorescence detecting modules 100, guides 331 and 332 which are installed at the frame 310, support the holder 310 to be moved and guide the movement, and a driver 340 which is installed at the frame 310 and allows the holder 320 to make a reciprocating motion.

Fluorescence dyes having various colors may be used in fluorescence detection in a real-time PCR reaction. One kind of fluorescence dye may be used in one microchamber 25 but various kinds of fluorescence dyes may be used together in one microchamber 25. In addition, different kinds of fluorescence dyes may also be used in each of a plurality of microchambers 25. In this case, the fluorescence detecting system 300 may have a plurality of fluorescence detecting modules 100 having wavelength selectivity so as to detect various fluorescence wavelengths. To this end, a plurality of, for example, six fluorescence detecting modules 100 may be installed in the holder 320 while being arranged in the same direction as the arrangement direction of the microchamber 25.

The guides 331 and 332 are long in the movement direction of the holder 320 and support the upper and lower portions of the holder 320. The driver 340 may comprise a lead screw 341 and a driving motor 342 which rotates the lead screw 341. The lead screw 341 is combined with a connection member 322 that is disposed in the holder 320 and allows the holder 320 and the fluorescence detecting module 100 to make a reciprocating motion due to its rotation. In the embodiment of the present invention, the pitch of the lead screw 341 is 3 mm and a rotation angle thereof is 18 degrees. The lead screw 341 is designed in 20 steps and the holder 320 is moved by 150µm per step.

The fluorescence detecting system 300 according to the present invention moves the fluorescence detecting module 100 along the arrangement direction of a plurality of microchambers 25 of the microfluid chip 10 and scans the fluorescence detecting module 100, thereby detecting fluorescence. In this case, a scanning distance must be more than a value that is the sum of the distance between optical axes of the first and last fluorescence detecting modules 100 and the overall width of the microfluid chip 10. For example, when the width of each of the fluorescence detecting modules 100 is 5.6 mm and the overall width of the microfluid chip 10 is 15 mm, the scanning distance must be more than 48.6 mm.

As described above, the fluorescence detecting system 300 according to the present invention comprises the fluorescence detecting module 100 shown in FIGS. 2 and 6 according to an embodiment of the present invention. However, it is obvious that the fluorescence detecting system 300 may comprise the fluorescence detecting module 200 shown in FIGS. 3 and 7 according to another embodiment of the present invention.

### <Experimental example >

Experiments for detecting fluorescence generated in the microchambers 25 of the microfluid chip 10 using the fluorescence detecting system 300 shown in FIG. 8 were carried out.

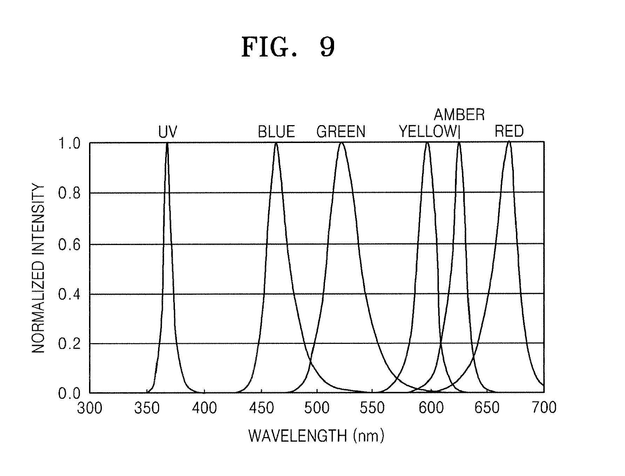

Six fluorescence detecting modules 100 were installed in the fluorescence detecting system 300, and six LEDs 110 for generating excitation light having different wavelengths were installed in the six fluorescence detecting modules 100. Wavelength spectrums of the LEDs 110 installed in the six fluorescence detecting modules 100 installed in the fluorescence detecting system 300 according to the present invention for the experiments is shown in FIG. 9. As shown in FIG. 9, the six LEDs 110 had wavelengths corresponding to ultraviolet (UV), blue, green, yellow, amber, and red.

Short-wavelength transmission filters each having a central wavelength of 390 nm, 495 nm, 545 nm, 610 nm, 645 nm, and 695 nm were used as the first filter 122 installed in the six fluorescence detecting modules 100. Long-wavelength transmission filters each having a central wavelength of 420 nm, 510 nm, 560 nm, 625 nm, 660 nm, and 710 nm were used as the second filter 128. Dichroic mirrors 124 each having a central wavelength of 400 nm, 505 nm, 555 nm, 620 nm, 655 nm, and 705 nm were used. When a distance between 10%T - 90%T transmission wavelengths is a filter width, short-wavelength transmission filters and long-wavelength transmission filters each having a filter width of less than 10 nm were used and the dichroic mirrors 124 each having a filter width of less than 20 nm were used.

JD1580 made by Juraron was used as the objective lens 126, and S1227-33BR made by Hamamatus was used as the photo diode 130. A current signal outputted from the photo diode 130 was converted into a voltage signal through an amplification circuit and was digitalized using an analog digital converter (ADC), and a current-to-voltage gain was measured to have a 1x10⁹ gain and was recorded by a computer.

A lower substrate 12 of the microfluid chip 10 used in experiments was manufactured by wet etching a silicon substrate having a thickness of 0.5 mm and by forming microchannels 23 and 24 and the microchambers 25, and an upper substrate 11 of the microfluid chip 10 was manufactured by forming a sample inlet 21 and a sample outlet 22 in a pyrex glass having a thickness of 0.5 mm using a sandblasting process. Eight microchambers 25 were formed in the lower substrate 12, and the distance between the microchambers 25 was 2 mm, the width of each of the microchambers 25 was 1.5 mm, and the depth of each of the microchambers 25 was 200 µm.

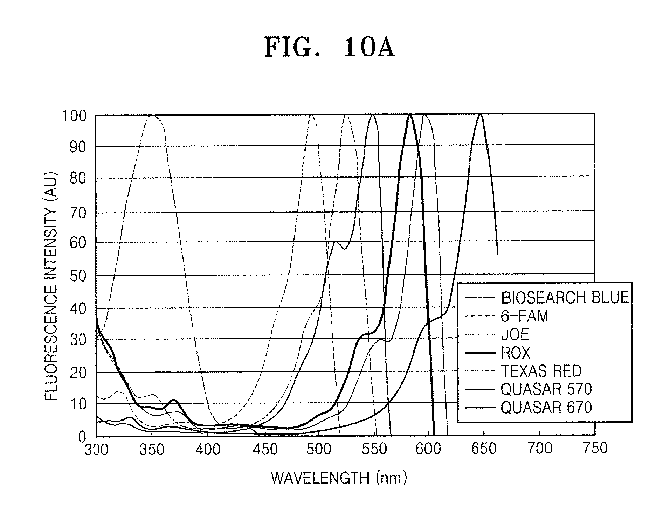

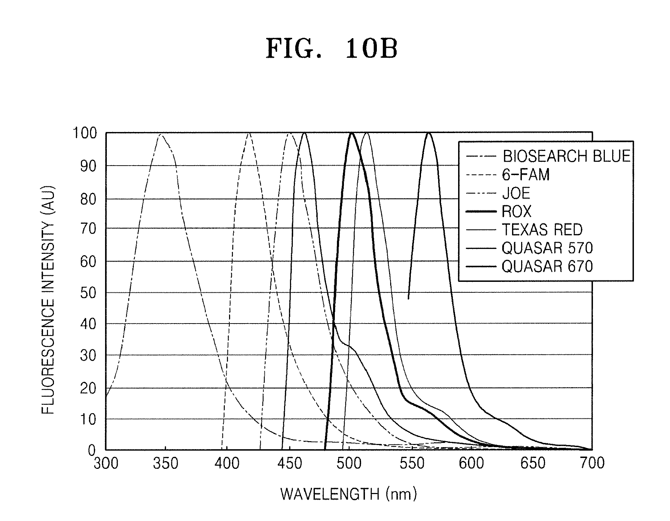

PH 9.6, 100 mM of a sodium borate buffer solution was injected into one of the eight microchambers 25, pH 7.6, 100 mM of TE buffer in which 100µM of 10T-oligonucleotide in which different kinds of fluorescence dyes were combined was injected into the other seven microchambers 25. Biosearch blue, 6-FAM, JOE, ROX, Texas Red, Quasar 570, and Quasar 670 were used as fluorescence dyes. FIG. 10A shows excitation spectrums of the fluorescence dyes, and FIG. 10B shows fluorescence spectrums of the fluorescence dyes.

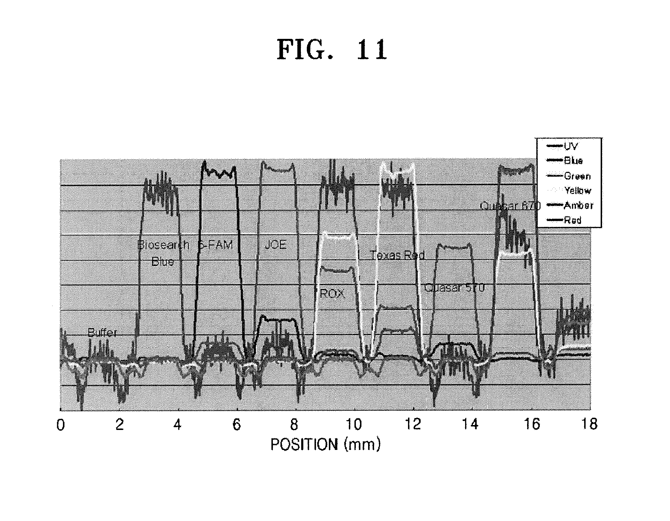

In the state where six fluorescence detecting modules 100 manufactured as described above are operated one by one, the fluorescence detecting modules 100 were oscillated at a maximum frequency of 3000 Hz in a 1/8 microstep and detected fluorescence generated in the microchambers 25. Fluorescence spectrums detected by the fluorescence detecting module 100 according to the present invention as a result of experiments is shown in FIG. 11. Referring to FIG. 11, six fluorescence detecting modules 100 which detect fluorescence having different wavelengths operated well.

As described above, in the fluorescence detecting module according to the present invention, the optical spot of excitation light irradiated on the microchambers is optimized, and even when the distance between a plurality of microchambers is narrower than less than 2 mm, excitation light does not affect the adjacent microchambers and fluorescence in a particular microchamber can be detected.

Furthermore, the fluorescence detecting module according to the present invention uses an LED and a photo diode, and a lens having a diameter of a clear aperture less than 4 mm such that the overall size of optical components is reduced, a fluorescence detecting module having a very small size is implemented, an optical path is reduced, the angle of optical components is reduced, and the size of an allowable error according to the angle of optical components and position tolerance is increased.

In addition, in the fluorescence detecting system according to the present invention, since the size of the fluorescence detecting module is reduced, a driving means is simple and becomes small, fluorescence is detected using a scanning method, and a fluorescence detecting time is reduced.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A fluorescence detecting module comprising:
a light source irradiating excitation light;
a collimating lens condensing excitation light irradiated from the light source;
a dichroic mirror selectively transmitting or reflecting the light according to its wavelength;
an objective lens condensing excitation light selected by the dichroic mirror to be irradiated on the sample in a microchamber and condensing fluorescence generated in the microchamber;
a focusing lens focusing fluorescence selected by the dichroic mirror; and
a fluorescence detecting element detecting fluorescence focused by the focusing lens.

2. The fluorescence detecting module of claim 1, wherein the light source is a light emitting diode (LED) having a surface emission shaped LED chip, and an emission surface of the LED chip is projected onto a sample in the microchamber as an optical spot having a predetermined area.

3. The fluorescence detecting module of claim 2, wherein the ratio of the area of the optical spot to the area of the emission surface of the LED chip is 1 or less than 1.

4. The fluorescence detecting module of claim 2, wherein the optical spot is positioned in the microchamber.

5. The fluorescence detecting module of claim 4, wherein the optical spot is positioned at the middle of the depth of the microchamber.

6. The fluorescence detecting module of claim 2, wherein the emission surface of the LED chip has a shape that is long in the lengthwise direction of the microchamber.

7. The fluorescence detecting module of claim 2, wherein the LED is an LED having no lens.

8. The fluorescence detecting module of claim 1, wherein the collimating lens condenses excitation light into substantially parallel light.

9. The fluorescence detecting module of claim 1, wherein the dichroic mirror is disposed to be inclined at approximately 45 degrees with respect to an optical axis of excitation light irradiated from the light source and selectively transmitting, or reflecting at right angles, excitation light and fluorescence according to their wavelengths.

10. The fluorescence detecting module of claim 9, wherein the dichroic mirror reflects short-wavelength components of excitation light at right angles to be directed toward the objective lens and transmits long-wavelength components of the fluorescence to be directed toward the focusing lens.

11. The fluorescence detecting module of claim 9, wherein the dichroic mirror transmits short-wavelength components of excitation light to be directed toward the objective lens and reflects long-wavelength components of the fluorescence at right angles to be directed toward the focusing lens.

12. The fluorescence detecting module of claim 1, wherein the fluorescence detecting element is a photo diode.

13. The fluorescence detecting module of claim 1, further comprising:
a first filter disposed between the collimating lens and the dichroic mirror and selecting a wavelength of excitation light; and
a second filter disposed between the dichroic mirror and the focusing lens and selecting a wavelength of fluorescence.

14. The fluorescence detecting module of claim 13, wherein the first filter is disposed at right angles with respect to an optical axis of excitation light irradiated from the light source, and the second filter is disposed at right angles with respect to an optical axis of fluorescence that is directed towards the fluorescence detecting element.

15. The fluorescence detecting module of claim 13, wherein the first filter is a short-wavelength transmission filter transmitting short-wavelength components of excitation light, and the second filter is a long-wavelength transmission filter transmitting long-wavelength components of fluorescence.

16. The fluorescence detecting module of claim 13, wherein the first filter and the second filter are dichroic filters.

17. The fluorescence detecting module of 1, further comprising a base in which a first optical path, a second optical path, and a third optical path connected to one another are formed, wherein excitation light irradiated from the light source is projected onto a sample in the microchamber through the first optical path and the second optical path, and fluorescence generated in the microchamber reaches the fluorescence detecting element through the second optical path and the third optical path.

18. The fluorescence detecting module of claim 17, wherein the light source is installed at an end of the first optical path, the objective lens is installed at an end of the second optical path, the fluorescence detecting element is installed at an end of the third optical path, the collimating lens is installed within the first optical path, and the focusing lens is installed within the third optical path, and
the dichroic mirror is inserted and installed in a position in which
the first optical path, the second optical path, and the third optical path meet one another to be inclined at approximately 45 degrees with respect to the optical axis of excitation light irradiated from the light source.

19. The fluorescence detecting module of claim 18, wherein the second optical path and the third optical path are parallel to each other in a vertical direction and the first optical path is formed in a horizontal direction and meets the second optical path and the third optical path at right angles.

20. The fluorescence detecting module of claim 19, wherein the dichroic mirror reflects short-wavelength components of excitation light that has passed through the first optical path at right angles to be directed toward the objective lens through the second optical path, and the dichroic mirror transmits long-wavelength components of fluorescence that is generated in the microchamber and has passed through the second optical path to be directed toward the focusing lens through the third optical path.

21. The fluorescence detecting module of claim 18, wherein the first optical path and the second optical path are parallel to each other in a vertical direction and the third optical path is formed in a horizontal direction and meets the first optical path and the second optical path at right angles.

22. The fluorescence detecting module of claim 21, wherein the dichroic mirror transmits short-wavelength components of excitation light that has passed through the first optical path to be directed toward the objective lens through the second optical path, and the dichroic mirror reflects long-wavelength components of fluorescence that are generated in the microchamber and that have passed through the second optical path at right angles to be directed toward the focusing lens through the third optical path.

23. The fluorescence detecting module of claim 18, wherein a first filter that selects a wavelength of excitation light between the collimating lens and the dichroic mirror is further installed in the first optical path, and a second filter that selects a wavelength of fluorescence between the focusing lens and the dichroic mirror is further installed in the third optical path.

24. The fluorescence detecting module of claim 23, wherein the first filter is a short-wavelength transmission filter disposed at right angles with respect to an optical axis of excitation light and transmitting short-wavelength components of excitation light, and the second filter is a long-wavelength transmission filter disposed at right angles with respect to an optical axis of fluorescence and transmitting long-wavelength components of fluorescence.

25. A fluorescence detecting system for a microfluid chip in which a plurality of microchambers are arranged, the system comprising:
a frame;
at least one fluorescence detecting module detecting fluorescence in the microchamber;
a holder supporting the at least one fluorescence detecting module;
a driver installed in the frame and allowing the holder to make a reciprocating motion along a direction in which the plurality of microchambers are arranged; and
a guide installed in the frame, supporting the holder to be moved and guiding the movement,
wherein the fluorescence detecting module comprises:
a light source irradiating excitation light;
a collimating lens condensing excitation light irradiated from the light source;
a dichroic mirror selectively transmitting or reflecting the light according to its wavelength;
an objective lens condensing excitation light selected by the dichroic mirror to be irradiated on the sample in a microchamber and condensing fluorescence generated in the microchamber;
a focusing lens focusing fluorescence selected by the dichroic mirror; and
a fluorescence detecting element detecting fluorescence focused by the focusing lens.

26. The fluorescence detecting system of claim 25, wherein a plurality of fluorescence detecting modules arranged in the same direction as the arrangement direction of the plurality of microchambers are installed in the holder.

27. The fluorescence detecting system of claim 26, wherein the plurality of fluorescence detecting modules detect at least two kinds of fluorescence having different wavelengths.

28. The fluorescence detecting system of claim 26, wherein each of the plurality of fluorescence detecting modules irradiates excitation light having different wavelengths and detects fluorescence having different wavelengths.

29. The fluorescence detecting system of claim 25, wherein the driver comprises a lead screw combined with the holder and a driving motor rotating the lead screw.

30. The fluorescence detecting system of claim 25, wherein the guide is long in the movement direction of the holder and supports upper and lower portions of the holder.

31. The fluorescence detecting system of claim 25, wherein the light source is a light emitting diode (LED) having a surface emission shaped LED chip, and an emission surface of the LED chip is projected onto a sample in the microchamber as an optical spot having a predetermined area.

32. The fluorescence detecting system of claim 2, wherein the ratio of the area of the optical spot to the area of the emission surface of the LED chip is 1 or less than 1.

33. The fluorescence detecting system of claim 31, wherein the LED is an LED having no lens.

34. The fluorescence detecting system of claim 25, wherein the dichroic mirror is disposed to be inclined at approximately 45 degrees with respect to an optical axis of excitation light irradiated from the light source and selectively transmits, or reflects at right angles, excitation light and fluorescence according to their wavelengths.

35. The fluorescence detecting system of claim 34, wherein the dichroic mirror reflects short-wavelength components of excitation light at right angles to be directed toward the objective lens and transmits long-wavelength components of the fluorescence to be directed toward the focusing lens.

36. The fluorescence detecting system of claim 34, wherein the dichroic mirror transmits short-wavelength components of excitation light to be directed toward the objective lens and reflects long-wavelength components of the fluorescence at right angles to be directed toward the focusing lens.

37. The fluorescence detecting system of claim 25, further comprising:
a first filter disposed between the collimating lens and the dichroic mirror and selecting a wavelength of excitation light; and
a second filter disposed between the dichroic mirror and the focusing lens and selecting a wavelength of fluorescence.

38. The fluorescence detecting system of claim 37, wherein the first filter is a short-wavelength transmission filter transmitting short-wavelength components of excitation light, and the second filter is a long-wavelength transmission filter transmitting long-wavelength components of fluorescence.

39. The fluorescence detecting system of claim 37, wherein the first filter ad the second filter are dichroic filters.

40. The fluorescence detecting system of 25, further comprising a base in which a first optical path, a second optical path, and a third optical path connected to one another are formed, wherein excitation light irradiated from the light source is projected onto a sample in the microchamber through the first optical path and the second optical path, and fluorescence generated in the microchamber reaches the fluorescence detecting element through the second optical path and the third optical path.

41. The fluorescence detecting system of claim 40, wherein the light source is installed at an end of the first optical path, the objective lens is installed at an end of the second optical path, the fluorescence detecting element is installed at an end of the third optical path, the collimating lens is installed within the first optical path, and the focusing lens is installed within the third optical path, and
the dichroic mirror is inserted and installed in a position in which
the first optical path, the second optical path, and the third optical path meet one another, to be inclined at approximately 45 degrees with respect to the optical axis of excitation light irradiated from the light source.
